# EUROPEAN PATENT APPLICATION

(11) **EP 4 306 957 A1**
(43) Date of publication of application: **17.01.2024**
(21) Application number: 22306034.4
(22) Date of filing: 11.07.2022
(51) Int. Cl.: G01N 33/543, G01N 33/50, G01N 33/53, G01N 33/551, G01N 33/94

(54) **METHOD FOR QUANTITATIVE MEASUREMENT OF ANTIBIOTICS, AND FLUORESCENT PROBES USED THEREIN**

(71) Applicant: Wainvam-E, 56270 Ploemeur (FR)
(72) Inventor: PERRE, Agathe, 29170 Pleuven (FR); LE GUEVELLO, Morgane, 56300 Malguénac (FR); RANI, Dipti, 56100 Lorient (FR)
(74) Representative: Novagraaf Technologies

(57) **Abstract**

The present invention relates to a method for quantitative measurement of antibiotics in a sample, comprising the steps of:
(1) Functionalizing nanodiamonds having Nitrogen-Vacancy (NV) centers with ligands able to bind specifically to said antibiotics,
(2) Bringing said functionalized nanodiamonds into contact with said antibiotics, under conditions allowing binding between said nanodiamond and said antibiotics,
(3) Illuminating the NV centers with green light, and
(4) Measuring red fluorescence emitted by the NV centers, wherein the amount of red fluorescence emitted by the NV centers is proportional to the amount of nanodiamonds bound to the antibiotics.

The present invention also relates to a carboxylated nanodiamond having Nitrogen-Vacancy (NV) centers, which is functionalized with monoclonal antibodies that are able to bind specifically to antibiotics, and to a device comprising a processor and memory, said memory comprising code that, when executed by said processor, causes the device to perform the method of the invention.

The present invention further relates to the use of the method as defined above, or of a functionalized nanodiamond as defined above, for quantitative measurement of antibiotics in the food industry, in water treatment, in aquaculture or in farming.

Finally, the invention relates to a method for preparing a carboxylated nanodiamond having Nitrogen-Vacancy (NV) centers, which is functionalized with monoclonal antibodies that are able to bind specifically to an antibiotic.

## Description

### Technical field

The present invention refers to a method for quantitative measurement of antibiotics in a sample, to functionalized nanodiamonds having Nitrogen-Vacancy (NV) centers, and to the use of the method or of the functionalized nanodiamonds for quantitative measurement of antibiotics.

Therefore, the present invention has utility in industry field, notably in farming, and in food industry.

In the description below, the reference into brackets ([]) refers to the Reference List situated at the end of the text.

### Background of the Invention

Foodstuffs intended for human consumption as meat or milk, contain traces of antibiotics from veterinary medicine in particular beta-lactamines, cyclines or sulfamides. These antibiotic residues can alter the health of consumers by inducing allergies and renal toxicity. They can also induce inhibition of biotechnological production processes such as lactic ferments for example in dairy product and they contribute to the phenomenon of antibioresistance.

Antibiotics are also a problem in water. Antibiotic residues are found in drinking water during wastewater treatment. These antibiotic residues contribute to the phenomenon of antibiotic resistance: the residues are found in nature and cause mutations in bacteria and viruses in the environment. In this context, it is possible to measure ciprofloxacin, an antibiotic that is present in greater quantities in water, using a rabbit monoclonal antibody (Huang et al.: "Preparation of high-affinity rabbit monoclonal antibodies for ciprofloxacin and development of an indirect competitive ELISA for residues in milk". Journal of Zhejiang University. Science. 2010. B, 11(10), 812-818 ([1])). Also, to measure vancomycin, it is possible to use monoclonal antibodies to quantify them (Fujiwara et al.: 'Immunocytochemistry for vancomycin using a monoclonal antibody that reveals accumulation of the drug in rat kidney and liver". Antimicrobial agents and chemotherapy, 2012, 56(11), 5883-5891 ([2]). These methods enable them to control antibiotics and thus prevent humans from curbing diseases.

Aquaculture, which has grown considerably in recent years, is also affected by antibiotics (Santos Lúcia, et Fernando Ramos. « Analytical strategies for the detection and quantification of antibiotic residues in Aquaculturefishes: A review ». Trends in Food Science & Technology 52 (1 avril 2016) ([3])). In aquaculture, antibiotics of macrolide and quinolone families are found. Industrially produced fish and seafood are prone to infections due to the large number of animals raised in confined spaces and their stressful farming and transport conditions. Eventually, this fish ends up on plates intended to humans who can develop resistance to antibiotics. It is therefore necessary to detect them beforehand in order to respect the limits.

Therefore, in water treatment plants and in aquaculture, there is a need for accurate antibiotic measurement tools to slow down the phenomenon of antibiotic resistance.

To protect the health of consumers and to anticipate issues in production, the 2017/880 European Commission regulation imposes a maximum residue limit for each of these antibiotics and for each given food.

Currently, Billon and Tao method (Billon, J., et Seng Huor Tao. « Detection des antibiotiques dans le lait. Identification et dosage par la méthode électrophorétique ». Le Lait 59, no 587 (1979): 361-75 ([4])) is used for antibiotic detection and is based on the diffusion of antibiotics from an inhibited disc of milk onto an agar plate inoculated with Bacillus *stearothermophilus.* Colorimetric methods are also used, such as DELVOTEST^{®} SP provided by Grosseron, based on the inhibition of the development of Bacillus stearothermophilus resulting in the absence of acidification revealed by a colored indicator. Billon and Tao ([4]) and DELVOTEST^{®} SP require skilled staff and create pollution by using chemical colorimetric product. Moreover, they only allow a qualitative measurement.

For other antibiotics as tetracycline and sulfonamide, a lateral flow assay (LFA) test (Li et al.: "Rapid Quantitative Detection for Multiple Antibiotics in Honey Using a Quantum Dot Microsphere Immunochromatographic Strip". Food Control 130, 1 December 2021, 108256 ([5])) has been developed using quantum dots that allow rapid quantification of the sample. The limit of detection is in the order of micrograms per kilogram. The disadvantages of this method are the use of quantum dots which have a high background detection level and the shells can alter the optical properties.

Thus, a need exists of a solution to quantify these antibiotics possibly at the farm gate, along the processing chain, and before to put products on the market to exclude foods with antibiotic residues above the current regulations. The present invention fulfills these and other needs.

### Description of the invention

After extensive research, the Applicant has managed to develop a solution for quantitative measurement of antibiotics.

Surprisingly, the Applicant has succeeded in developing a quantitative method of measurement of antibiotics by labeling with fluorescent nanodiamonds.

Advantageously, the Applicant made nanodiamonds having on their surfaces chemicals or biomolecules that can target antibiotics. These functionalized nanodiamonds can therefore mark and recognize specifically the antibiotics. Once the nanodiamonds are functionalized, they bind to antibiotics and, thanks to the fluorescence property of the nanodiamonds used, a quantitative measurement of antibiotics is then possible.

One of the advantages of the invention is that the measurement can be the long-term stability of fluorescence signal of nanodiamonds. Another advantage is that the technology is sensitive, accurate and capable of measuring all the antibiotics, unlike the techniques of prior art mentioned above. Moreover, the invention allows a measurement on the field, point of interest, for example in a farm, and at the earliest possible time during the beginning of processing line.

Another advantage of the invention is that it allows rapid measurement of antibiotics. Also, it can be used at room temperature (i.e. about 20-25°C).

Moreover, it is a specific method for the measurement of antibiotics that allows measuring all of the antibiotics used in veterinary and medicine simultaneously but also in a distinct way, depending on the functionalization of the nanodiamond chosen.

Accordingly, in a first aspect, the present invention provides a method for quantitative measurement of at least one antibiotic in a sample, comprising the steps of:
(1) Functionalizing nanodiamonds having Nitrogen-Vacancy (NV) centers with ligands able to bind specifically to said at least one antibiotic,
(2) Bringing said functionalized nanodiamonds into contact with said at least one antibiotic, under conditions allowing binding between said nanodiamond and said at least one antibiotic,
(3) Illuminating the NV centers with green light, and
(4) Measuring red fluorescence emitted by the NV centers, wherein the amount of red fluorescence emitted by the NV centers is correlated with the amount of nanodiamonds bound to the at least one antibiotic.

"Antibiotic(s)" refers herein to any antibiotic, for example at least one antibiotic selected among the class of penicillins, macrolides, cephalosporins, fluoroquinolones, beta-lactams, cyclins such as tetracyclin, polymyxins such as colistin, trimethoprim-sulfamethoxazole, urinary anti-infectives, lincosamides, glycopeptides, and sulfonamides. Especially, the invention allows to measure several antibiotics simultaneously or in a distinct way, depending on the functionalization of the nanodiamonds chosen.

"Nanodiamonds" refers herein to diamonds having nanoscale. Advantageously, the nanodiamonds may have a size included between 50nm and 150nm, or 70nm and 100 nm.

Optionnally, the nanodiamonds used in step (1) may be carboxylated. "Carboxylated" refers herein to a carboxyl group at the surface of nanodiamonds, that are able to be functionalized using carbodiimide chemistry. For example, carboxyl group may be present at the surface of fluorescent nanodiamonds due to chemical treatment.

"Nitrogen-Vacancy (NV) centers" refers herein to defects that are artificially created in nanodiamonds during their preparation. A carbon atom in the diamond's crystal lattice is replaced by a nitrogen atom (defect, N), and a neighboring lattice site is left empty (vacancy, V) to form NV center. These defects exhibit red fluorescence properties under green light excitation. As further explained below, the fluorescence properties of nanodiamonds with NV centers may be processed and quantitatively linked to antibiotic concentration.

"Functionalizing" refers herein to the linkage of chemicals or biomolecules at the surface of the nanodiamonds. Advantageously, functionalization of nanodiamonds make them able to recognize and link specifically to an antibiotic. Advantageously, functionalization of the surface carboxyl groups of nanodiamonds may be realized by any method known in the art for this purpose, as for example described in reference [1] for quantum dots. For example, in a first step, carboxylated groups on the nanodiamonds may be activated using 1-ethyl-3-(-3-dimethylaminopropyl) carbodiimide hydrochloride (EDC) and Sulfo-N-hydroxysuccinimide (Sulfo-NHS). The activated carboxyl groups are made to react with NH₂ terminated ligand or amine part of the ligand able to bind specifically to antibiotics to form ligand-nanodiamond via amide linkage between the carboxyl and amine groups. When an antibiotic of interest is made to react with ligand functionalized nanodiamonds, it results in the attachment of said antibiotic to the surface of the nanodiamonds, and thus the presence of antibiotic can be detected by using nanodiamond as a label.

"Ligands able to bind specifically to at least one antibiotic" refers herein to any chemicals or biomolecules known in the art to link at least one antibiotic. It may be for example aptamers or antibodies, which can be monoclonal or polyclonal antibodies specific of the antibiotic. In the case of tetracycline and sulfonamide detection, the surface of the nanodiamonds can be functionalized with monoclonal anti-tetracycline and monoclonal anti-sulfonamide antibodies respectively ([5]). Concerning β-lactams, it is possible to functionalize the nanodiamond with an anti-β-lactamase monoclonal antibody (Wang et al.: « Detection of β-Lactamase Residues in Milk by Sandwich ELISA ». International Journal of Environmental Research and Public Health 10, no 7 (1 June 2013): 2688 98 ([6])). In the case of colistin detection, it is possible to functionalize the nanodiamonds with an anti-colistin monoclonal antibody (Wang et al. « Rapid one-step enzyme immunoassay and lateral flow immunochromatographic assay for colistin in animal feed and food ». Journal of Animal Science and Biotechnology 10, no 1 (17 October 2019): 82 ([7]). Regarding macrolide family, it is possible to functionalise the nanodiamonds with anti-BSA-9-(carboxymethyloxime)-clarithromycin antibodies as in used by Raysyan et al. (Raysyan et al.: « Development of a Latex Particles-Based Lateral Flow Immunoassay for Group Determination of Macrolide Antibiotics in Breast Milk ». Journal of Pharmaceutical and Biomedical Analysis 189 (10 September 2020): 113450 ([8])). Regarding the quinolone family, it is possible to functionalize the nanodiamonds with monoclonal norfloxacin antibodies such as those described by Shi et al. (Shi et al.: « A SERS-based multiple immuno-nanoprobe for ultrasensitive detection of neomycin and quinolone antibiotics via a lateral flow assay ». Microchimica Acta 185 (January 2018) ([9])).

The step (2) of bringing said functionalized nanodiamonds into contact with said antibiotics may be realized by any method known by the skilled person for this purpose. This can be performed e.g. *in vitro* in a plate pre-functionalized with capture antibodies for a specific antibiotic, for example by incubating the functionalized nanodiamonds in a medium containing the antibiotic. Period and conditions of culture may be determined by the skilled person in view of his technical skills.

Step (3) of illuminating the NV centers with green light may be realized at a wavelength of between 480 and 600 nm. Advantageously, the red fluorescence emitted by NV centers in nanodiamonds is modulated with an electromagnet, in order to improve the limit of detection. The skilled person can choose according to his common knowledge in this technical field an optimized modulation frequency and numerical filter, in order to get rid of the continuous component of the autofluorescence. Then, the measurement may be advantageously more sensitive for nanodiamonds fluorescence and then for analyte amount.

Nanodiamonds bound to antibiotics may be detected by measuring red fluorescence emitted by the NV centers. Red fluorescence may be measured by any mean known in the state of the art, for example with an optical detector.

In an aspect of the invention, the functionalized nanodiamonds may be incorporated into an immunodiagnostic membrane. This immunodiagnostic membrane may be a strip, especially commonly used self-test type. In this purpose, the strip may be placed into a cassette, as those commonly used for these kinds of test, which contains a zone able to receive the drops of sample, and a zone wherein the sample is visible and able to migrate.

The strip may be any strip known in the state of the art for this purpose, as for example a lateral flow strip.

Advantageously, the strip may comprise the areas commonly found on such self-test.

It may be notably a sample drop area (also called "the sample pad"), an area in which the functionalized nanodiamonds are incorporated (also called "the conjugate pad"), a detection area (also called "nitrocellulose pad" or "NC membrane") and an absorbent area (also called "the absorbent pad").

Advantageously, the nanodiamonds bound to the antibiotics migrate along the strip to the detection area, after the sample containing antibiotics is deposited on the sample drop area.

The detection area generally comprises a test line and a control line.

In an embodiment of the invention, also called "competitive LFA", the detection area may comprise a test line and a control line, including respectively antibiotics and antibodies specific of functionalized nanodiamonds. In this embodiment, the extract of the test sample is placed on the sample pad. On this strip, the control line, comprising antibodies specific of the functionalized nanodiamond, allows to validate the migration of functionalized nanodiamonds. There are two possibilities of result. If the test is positive, then the sample contains antibiotics. The antibiotics bind to the functionalized nanodiamonds on the conjugate pad. Nanodiamonds coupled to antibiotic migrate along the strip to the detection area, illustrated by the NC membrane. As nanodiamonds are already linked to antibiotics, they can't link in the test line because on the test line there is antibiotics. So, the test line is not colored after laser passage. On the contrary, if the test is negative, then the sample doesn't contain antibiotic. When a drop of sample is put on the sample pad, nothing can link with functionalized nanodiamonds. The nanodiamond migrate along the strip to the detection area, illustrated by the NC membrane. As nanodiamond aren't linked to antibiotics, they can link in the test line and the test line is colored after laser illumination. As each functionalized nanodiamond is bound to antibiotic molecules, the amount of red fluorescence emitted is inversely proportional to the amount of nanodiamonds bound to the antibiotics, therefore allowing quantitative measurement of antibiotics.

In another embodiment, also called "sandwich LFA", the detection area may comprise a test line and a control line, including respectively antibodies specific of the antibiotics and antibodies specific of functionalized nanodiamonds. In this embodiment, the extract of the test sample is placed on the sample pad. On this strip, the control line allows to validate the migration of functionalized nanodiamonds as it comprises antibodies specific of the functionalized nanodiamond. On the test line, there are already specific antibiotic antibody. Therefore if there are antibiotics in the sample, they can link to nanodiamonds surface in the conjugate pad, and then link to the antibody in the test line, causing coloration of the test line. As each functionalized nanodiamond is bound to antibiotic molecules, the amount of red fluorescence emitted is proportional to the amount of nanodiamonds bound to the antibiotics, therefore allowing quantitative measurement of antibiotics.

The absorbent pad contains a highly absorbent material to prevent the leakage of remaining sample crossing the control line from the strip.

Advantageously, the functionalized nanodiamonds, eventually bound to the antibiotics, migrate by capillarity along the strip to the detection area, after the sample containing antibiotics is deposited on the sample drop area.

Advantageously, the detection area may be illuminated by a green laser with a wavelength between 480 nm and 600 nm, in order to allow quantitative measurement of antibiotics.

Advantageously, step (4) of measuring red fluorescence emitted by the NV centers, may be realized by any known method. For example, the result may appear on a screen, and as the fluorescence of test line is linked to the quantity of antibiotics molecules functionalised on nanodiamonds, a correlation curve may be made using different concentrations of the test analyte (i.e antibiotic) with intensity of the test line and amount of antibiotic. When a sample is tested with an unknown amount of antibiotic, the fluorescence may be measured and plotted on the calibration curve to determine the amount of analyte (i.e antibiotic) present in the sample.

In a further aspect, the present invention provides a carboxylated nanodiamond having Nitrogen-Vacancy (NV) centers, which is functionalized with antibodies that are able to bind specifically to said antibiotics, as defined above.

In another aspect, the present invention provides a device comprising a processor and memory, said memory comprising code that, when executed by said processor, causes the device to perform the method of the invention.

When provided by a processor, the functions may be provided by a single dedicated processor, by a single shared processor, or by a plurality of individual processors, some of which may be shared. Moreover, explicit use of the term "processor" should not be construed to refer exclusively to hardware capable of executing software, and may implicitly include, without limitation, digital signal processor (DSP) hardware, network processor, application specific integrated circuit (ASIC), field programmable gate array (FPGA), Graphical Processing Unit (GPU), read only memory (ROM) for storing software, random access memory (RAM), and non-volatile storage. Other hardware, conventional or custom, may also be included. Their function may be carried out through the operation of program logic, through dedicated logic, through the interaction of program control and dedicated logic, or even manually, the particular technique being selectable by the implementer as more specifically understood from the context.

According to the invention, memory may include or store computer program code, and the memory and the computer program code may be configured to, with the processor, cause a network element or network device to perform the necessary tasks. Additionally, the processor, memory and example algorithms, encoded as computer program code, serve as means for providing or causing performance of operations discussed herein.

In another aspect, the present invention provides the use of the method of the invention, or of a functionalized nanodiamond of the invention, for quantitative measurement of antibiotics in the food industry, in water treatment, in aquaculture or in farming.

In another aspect, the present invention provides a method for preparing a carboxylated nanodiamond having Nitrogen-Vacancy (NV) centers, which is functionalized with monoclonal antibodies that are able to bind specifically to an antibiotic, comprising the steps of:
(A) activating the carboxylated groups on the nanodiamonds using 1-ethyl-3-(-3-dimethylaminopropyl) carbodiimide hydrochloride (EDC) and sulfo-N-hydroxysuccinimide (sulfo-NHS),
(B) reacting the activated carboxyl groups with NH₂ part of antibody to bind an antibody to nanodiamond via amide linkage between
   the carboxyl and amine groups,
   thereby obtaining said functionalized nanodiamond.

This invention is further illustrated by the following examples with regard to the annexed drawings that should not be construed as limiting.

### Brief description of the figures

- Figure 1: represents an illustration of a (A) method for the functionalization of nanodiamonds in order to recognize antibiotics and (B) a binding of antibiotic to antibody-FND.
- Figure 2: represents a strip with the different pads: the 'sample pad' (also called "sample drop area" above), the conjugate pad (which is the area in which the functionalized nanodiamonds are incorporated), the nitrocellulose membrane (NC Membrane, also possibly called "detection area" above), and the absorbent pad (also called "absorbent area above"). The sample to be analyzed for the presence or absence of antibiotics is deposited in the 'sample pad' and migrates along the strip.
- Figure 3: represents an illustration of the principle of the competitive LFA test for antibiotic detection and of its result: when the biological sample to be tested contains the target antibiotic, only the control line is colored (positive test).
- Figure 4: represents an illustration of the principle of the competitive LFA test for antibiotic detection. When the biological sample to be tested does not contain the targeted antibiotic, the control line and the test line will emit a fluorescence (negative test).
- Figure 5: represents the antibiotics called C, link to the functionalized nanodiamond with specific antibody and after, the complex functionalized nanodiamond with the antibiotics can be revealed.
- Figure 6: represents a modulated magnetic field as well as a green laser, to which are subjected the nanodiamonds bound to antibiotics.
- Figure 7: represents a calibration curve, allowing to determine the concentration of antibiotics in the sample, which is correlated with the fluorescence intensity given once the test has been run through the reader.

### Examples

### Example 1: fluorescent nanodiamonds (FND) functionalization with monoclonal norfloxacin antibodies

Monoclonal antibodies binding specifically to norfloxacin are realized as described Shi et al. ([9]).

The antibody has a amine group (NH₂) at the end so it is functionalized onto carboxylated nanodiamond using carbodiimide chemistry.

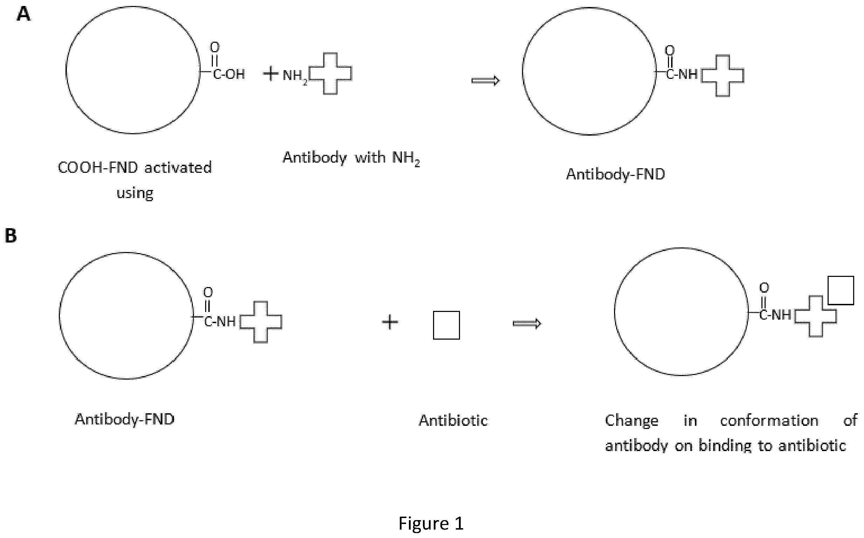

In the first step, the carboxylated groups onto nanodiamonds are activated with 1-ethyl-3-(3-dimethylaminopropyl) carbodiimide hydrochloride (EDC) and N-hydroxysulfosuccinimide sodium (sulfo-NHS). The activated carboxyl groups are made to react with NH₂ terminated antibody to form FND-antibody via amide linkage between the carboxyl and amine groups, as schematically illustrated in Figure 1.

When analyte of interest, norfloxacin, is made to react with antibody functionalized FNDs, the presence of norfloxacin can be detected by using FND as a label.

### Example 2: incorporation of the nanodiamonds previously functionalized into a lateral flow strip

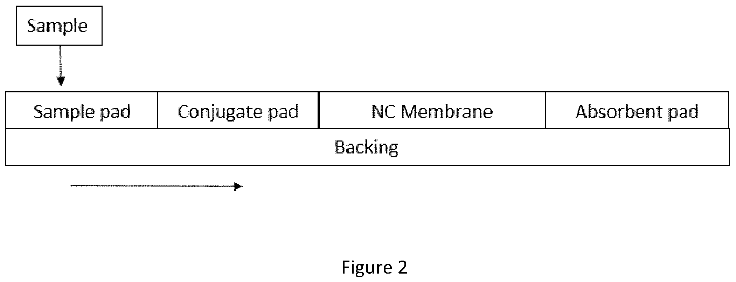

The nanodiamonds previously functionalized in the previous step are incorporated into a lateral flow strip composed of nitrocellulose as shown in Figure 2, on the conjugate pad before the beginning of the analysis.

Then, the sample likely to contain antibiotics is deposited in the first part of the strip called 'sample pad' (also called "sample drop area" above) and migrates along the strip. On this strip, it exists a test line and a control line to validate the migration of functionalized nanodiamonds. On the test line, there are already specific antibiotics. The control line comprises goat anti-mouse IgG (specific of the functionalized nanodiamond). There are two possibilities of result.

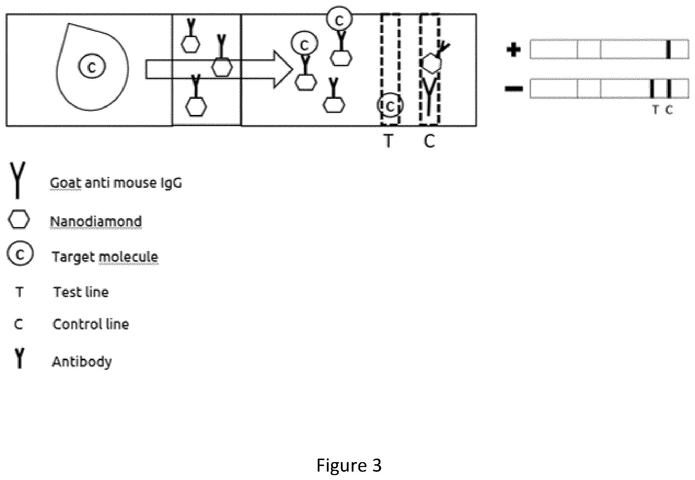

If it's a positive test : The sample contains antibiotics. They will bind to the previously functionalized nanodiamonds on the conjugate pad. Nanodiamonds coupled to antibiotic will migrate along the strip to the detection area, illustrated by the NC membrane. As nanodiamonds are already linked to antibiotics, they can't link in the test line because on the test line there are antibiotics. So, the test line is not colored after laser passage (see Figure 3).

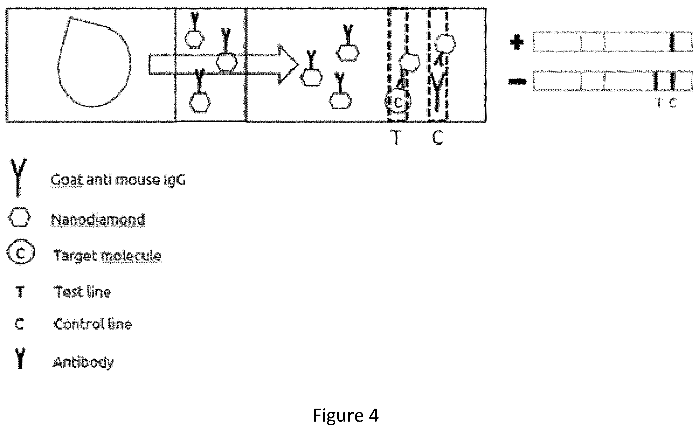

If it's a negative test: The sample doesn't contain antibiotic. When we put a drop in the sample pad, nothing can link with functionalized nanodiamonds. They will migrate along the strip to the detection area, illustrated by the NC membrane. As nanodiamond aren't linked to antibiotics, they can link in the test line and the test line is colored after laser illumination (see Figure 4).

### Example 3: incorporation of the nanodiamonds previously functionalized into a lateral flow strip

The nanodiamonds previously functionalized in the previous step are incorporated into a lateral flow strip composed of nitrocellulose as shown in Figure 2.

Then, the sample likely to contain antibiotics is deposited in the first part of the strip called 'sample pad' (also called "sample drop area" above) and migrates along the strip. If present, the antibiotics couple to the functionalized nanodiamonds present in the conjugate pad (which is the area in which the functionalized nanodiamonds are incorporated). Nanodiamonds coupled to antibiotics continue to migrate along the strip to the detection area, illustrated by the NC membrane on Figure 3.

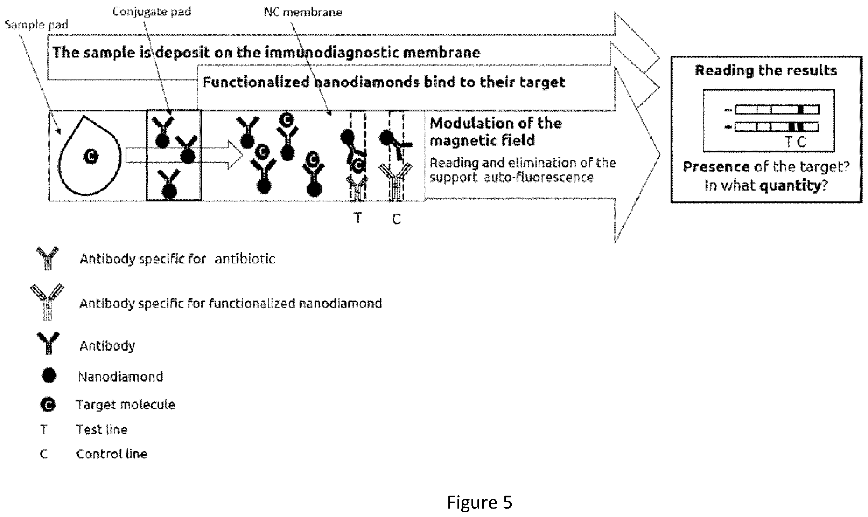

On this strip, it exists a test line comprising antibodies specific to antibiotics in order to check the presence of antibiotics and a control line to validate the migration of functionalized nanodiamonds, as shown in Figure 5. The ligand functionalized nanodiamond once bound to the antibiotic, it migrates along the detection area, and the complex is captured at the test line, which indicates the presence of antibiotic in the test sample. The antibodies on the control line (specific of the functionalized nanodiamond), are immobilized only to confirm the flow if the flow of the functionalized nanodiamonds worked well, independent of the presence/absence of antibiotic in the sample.

The absorbent pad, also called "absorbent area", is also represented.

### Example 4: Quantitative measurement of antibiotics

To read the result and the amount of antibiotics in the sample as previously tested with a test strip, the strip in the form of a cassette is put on equipment. Then, the strip receives the laser and the magnetic field.

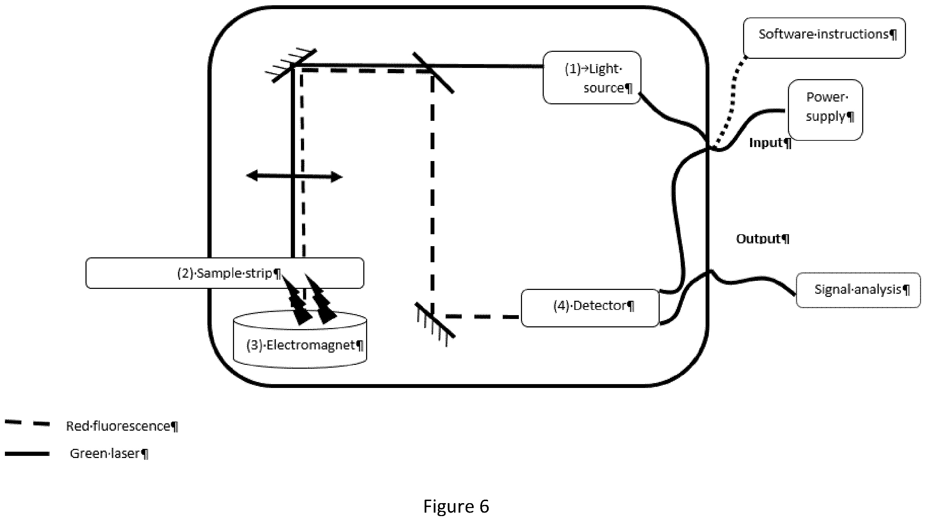

The detection area is illuminated by a green laser with a wavelength between 480 nm and 600 nm. Then, nanodiamonds illuminated by green light, emit red light as shown in Figure 6. To improve the limit of detection, the fluorescence signal is modulated with an electromagnet as shown in this figure. By choosing the right modulation frequency and a numerical filter, it is possible to get rid of the continuous component of the autofluorescence caused by the NC membrane. Then, the measurement is more sensitive for nanodiamonds fluorescence and then for analyte amount.

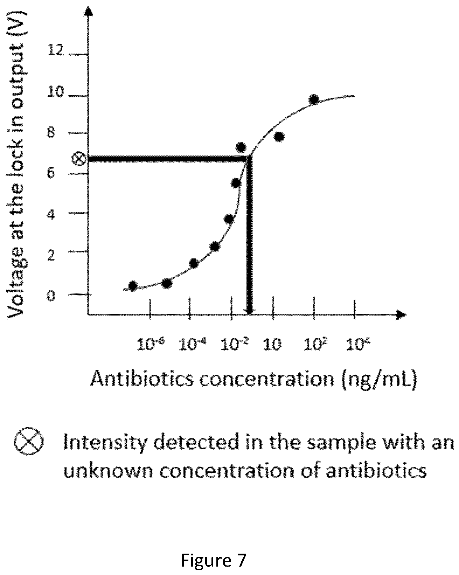

After that, the result appears on a screen as on figure (Fig.7). The fluorescence test line is linked to the quantity of antibiotics molecules functionalised on nanodiamonds. A correlation curve is made using different concentrations of the test analyte (i.e antibiotic) (on the x-axis) with intensity of the test line (on the y-axis). When a sample is tested with an unknown amount of antibiotic, the fluorescence can be measured and plotted on the calibration curve to determine the amount of analyte (i.e antibiotic) present in the sample, and is proportional to the amount of nanodiamonds bound to the antibiotics in the case of sandwich LFA, as each functionalized nanodiamond binds to antibiotics, and inversely proportional in the case of competitive LFA.

Therefore, the fluorescence test line of nanodiamonds is linked to the quantity of antibiotics molecules in the sample.

### Reference List

1. Huang et al.: "Preparation of high-affinity rabbit monoclonal antibodies for ciprofloxacin and development of an indirect competitive ELISA for residues in milk". Journal of Zhejiang University. Science. 2010. B, 11(10), 812-818.
2. Fujiwara et al.: 'Immunocytochemistry for vancomycin using a monoclonal antibody that reveals accumulation of the drug in rat kidney and liver". Antimicrobial agents and chemotherapy, 2012, 56(11), 5883-5891
3. Santos, Lúcia, et Fernando Ramos. « Analytical strategies for the detection and quantification of antibiotic residues in Aquaculturefishes: A review ». Trends in Food Science & Technology 52 (1 avril 2016).
4. Billon, J., et Seng Huor Tao. « Detection des antibiotiques dans le lait. Identification et dosage par la méthode électrophorétique ». Le Lait 59, no 587 (1979): 361-75.
5. Li et al.: "Rapid Quantitative Detection for Multiple Antibiotics in Honey Using a Quantum Dot Microsphere Immunochromatographic Strip". Food Control 130. 1 decembre 2021, 108256.
6. Wang et al.: « Detection of β-Lactamase Residues in Milk by Sandwich ELISA ». International Journal of Environmental Research and Public Health 10, no 7 (1 juin 2013): 2688 98.
7. Wang et al. « Rapid one-step enzyme immunoassay and lateral flow immunochromatographic assay for colistin in animal feed and food ». Journal of Animal Science and Biotechnology 10, no 1 (17 October 2019): 82.
8. Raysyan et al.: « Development of a Latex Particles-Based Lateral Flow Immunoassay for Group Determination of Macrolide Antibiotics in Breast Milk ». Journal of Pharmaceutical and Biomedical Analysis 189 (10 September 2020): 113450.
9. Shi et al.: « A SERS-based multiple immuno-nanoprobe for ultrasensitive detection of neomycin and quinolone antibiotics via a lateral flow assay ». Microchimica Acta 185 (January 2018).

## Claims

1. A method for quantitative measurement of at least one antibiotic in a sample, comprising the steps of:
(1) Functionalizing nanodiamonds having Nitrogen-Vacancy (NV) centers with ligands able to bind specifically to said at least one antibiotic,
(2) Bringing said functionalized nanodiamonds into contact with said at least one antibiotic, under conditions allowing binding between said nanodiamond and said at least one antibiotic,
(3) Illuminating the NV centers with green light, and
(4) Measuring red fluorescence emitted by the NV centers, wherein the amount of red fluorescence emitted by the NV centers is correlated with the amount of nanodiamonds bound to the at least one antibiotic.

2. A method according to claim 1, wherein said at least one antibiotic are at least one antibiotic among the class of penicillins, macrolides, cephalosporins, fluoroquinolones, beta-lactams, cyclins, polymyxins, trimethoprim-sulfamethoxazole, urinary anti-infectives, lincosamides, glycopeptides, and sulfonamides.

3. A method according to any of claim 1 or 2, wherein the functionalized nanodiamonds are incorporated into a strip, for example a lateral flow strip.

4. A method according to claim 3, wherein the strip comprises a sample drop area, an area in which the functionalized nanodiamonds are incorporated, a detection area and an absorbent area.

5. A method according to claim 4, wherein the nanodiamonds bound to the antibiotics migrate along the strip to the detection area, after the sample containing at least one antibiotic is deposited on the sample drop area.

6. A method according to any of claims 4 or 5, wherein the detection area comprises a test line and a control line, including respectively antibodies specific of the at least one antibiotic and antibodies specific of functionalized nanodiamonds.

7. A method according to any of claims 4 or 5, wherein the detection area comprises a test line and a control line, including respectively at least one antibiotic and antibodies specific of functionalized nanodiamonds.

8. A method according to any of claims 4 to 7, wherein the detection area is illuminated by a green laser with a wavelength between 480 nm and 600 nm.

9. A method according to claim 6, wherein the amount of red fluorescence emitted by the strip is proportional to the amount of nanodiamonds bound to the at least one antibiotic.

10. A method according to claim 7, wherein the amount of red fluorescence emitted by the strip is inversely proportional to the amount of nanodiamonds bound to the at least one antibiotic.

11. A method according to anyone of the preceding claims, wherein ligands are monoclonal or polyclonal antibodies specific of the at least one antibiotic.

12. A carboxylated nanodiamond having Nitrogen-Vacancy (NV) centers, which is functionalized with monoclonal or polyclonal antibodies that are able to bind specifically to antibiotics.

13. A device comprising a processor and memory, said memory comprising code that, when executed by said processor, causes the device to perform the method according to any one of the claims 1 to 11.

14. Use of a method as defined in any of claims 1 to 11, or of a functionalized carboxylated nanodiamond as defined in claim 12, for quantitative measurement of antibiotics in the food industry, in water treatment, in aquaculture or in farming.

15. A method for preparing a carboxylated nanodiamond having Nitrogen-Vacancy (NV) centers, which is functionalized with monoclonal antibodies that are able to bind specifically to an antibiotic, comprising the steps of:
(A) activating the carboxylated groups on the nanodiamonds using 1-ethyl-3-(-3-dimethylaminopropyl) carbodiimide hydrochloride (EDC) and sulfo-N-hydroxysuccinimide (sulfo-NHS),
(B) reacting the activated carboxyl groups with NH₂ part of antibody to bind an antibody to nanodiamond via amide linkage between
the carboxyl and amine groups,
thereby obtaining said functionalized nanodiamond.
